# EUROPÄISCHE PATENTSCHRIFT

(11) **EP 1 138 287 B1**
(45) Veröffentlichungstag und Bekanntmachung des Hinweises auf die Patenterteilung: **15.09.2004**
(21) Anmeldenummer: 01100779.6
(22) Anmeldetag: 13.01.2001
(51) Int. Cl.: A61F 5/01

(54) **Fussheberorthese**
Drop foot orthosis
Orthèse pour pied tombant

(30) Priorität: 28.03.2000 DE 20005737 U
(43) Veröffentlichungstag der Anmeldung: 04.10.2001
(73) Patentinhaber: Otto Bock HealthCare GmbH, 37115 Duderstadt (DE)
(72) Erfinder: Wellershaus, Ulf, 37115 Duderstadt (DE); Vollbrecht, Matthias, 37412 Herzberg (DE)
(74) Vertreter: Gramm, Werner, Prof. Dipl.-Ing.

(56) Entgegenhaltungen:
- DE-A- 3 916 091
- DE-C- 3 928 627
- US-A- 3 916 886
- US-A- 5 853 380
- US-A- 5 897 515
- US-A- 6 146 344

## Beschreibung

Die Erfindung betrifft eine Fußheberorthese mit einer dorsalen, sich über den Rückfuß erstreckenden, aus Kunststoff bestehenden Beinschale, die über eine Gurtung oder dergleichen an der Wade des Orthesenträgers festlegbar ist und sich mit einem mit ihr einteilig ausgebildeten Vorfuß-Schalenabschnitt bis unter den Fußsohlenbereich erstreckt.

Eine hinsichtlich der Beinschale ähnlich ausgebildete, als Sprunggelenkorthese konzipierte Fußorthese ist in der EP 0 567 783 B1 oder US-A-5 853 380 beschrieben.

Der Erfindung liegt die Aufgabe zugrunde, die eingangs beschriebene Fußheberorthese hinsichtlich ihrer Anpassung an den Fuß des Orthesenträgers und an das vom Orthesenträger getragene Schuhwerk zu verbessern.

Ausgehend von der eingangs definierten Fußheberorthese wird diese Aufgabe erfindungsgemäß dadurch gelöst, dass die Beinschale in dorsaler Fußbeugung einen geringen und in plantarer Fußbeugung einen hohen, die Fuß- und Schuhmasse kompensierenden Widerstand aufweist und aus einem weichen, biegeelastischen Kunststoff, z.B. einem Low Density Polyethylen oder einem EVA, besteht, in den auf der Rück- bzw. Unterseite der Beinschale zwei Federstahl-Drahteinlagen eingebettet sind, die sich angenähert parallel zu und in geringem Abstand von dem Innen- bzw. Außenrand der Beinschale von deren Wadenbereich bis unter den Vorfuß-Schalenabschnitt erstrecken.

Der Verlauf der Drahteinlagen bestimmt die wesentlichen Eigenschaften der Orthese. Dabei ist es zweckmäßig, wenn die Drahteinlagen etwa im unteren Bereich des Fersenbeines auslaufen.

Zur Verbesserung des Tragekomforts ist es vorteilhaft, wenn die Drahteinlagen nahezu vollständig vom Kunststoff umschlossen sind.

Zur Schonung des Schuhrandes eines vom Orthesenträger angelegten Schuhwerkes ist es vorteilhaft, wenn eine mittlere, im hinteren Fersenbereich der Beinschale auf deren Rückseite angeformte Rippe vorgesehen ist, die bei angelegtem Schuhwerk über dessen Schuhrand hinausragt.

Als Kunststoff eignet sich insbesondere z.B. ein Low Density Polyethylen LD PE 100, der weich genug ist, um sich dem Fuß des Orthesenträgers sowie dem angelegten Schuhwerk ohne manuelle Zurichtung selbsttätig anzupassen. Erfindungsgemäß wird somit eine autoadaptive Fußorthese geschaffen, deren Drahteinlagen plantar eine feste Orthesenausbildung sicherstellen. Verwendet wird vorzugsweise ein vorgebogener Federstahldraht von 1,5 bis 2 mm Durchmesser, der vor dem Tiefziehen der Beinschale auf Distanzhalter fixiert wird, um eine vollständige Umschließung von dem Kunststoff zu erzielen. Eine Herstellung der Beinschale im Spritzgießverfahren ist grundsätzlich möglich.

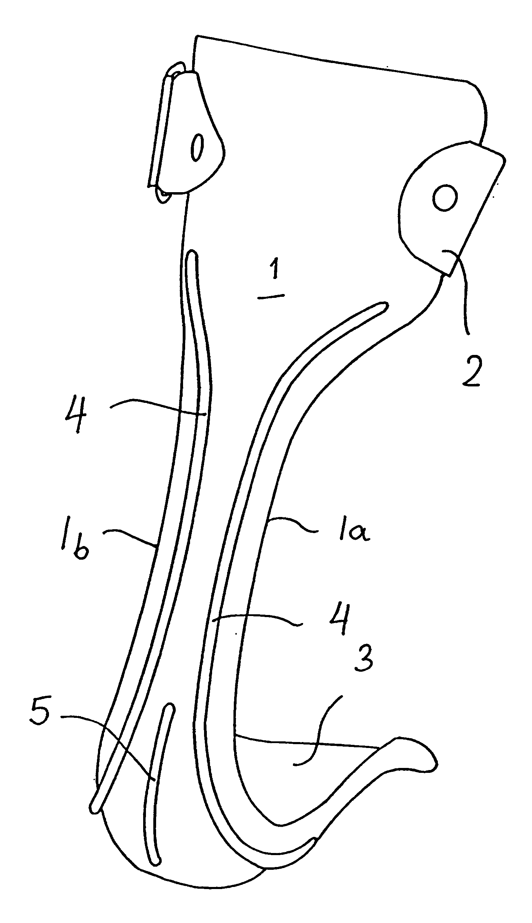

In der Zeichnung ist eine als Beispiel dienende Ausführungsform der Erfindung dargestellt.

Die Zeichnung zeigt in Rückansicht eine Fußheberorthese mit einer dorsalen, sich über den Rückfuß erstreckenden, aus Kunststoff bestehenden Beinschale 1, die über eine Gurtung 2, die z.B. ein Klettbandverschluss sein kann, an der Wade des Orthesenträgers festlegbar ist.

Die Beinschale 1 erstreckt sich mit einem mit ihr einteilig ausgebildeten Vorfuß-Schalenabschnitt 3 bis unter den Fußsohlenbereich und weist auf ihrer Rück- bzw. Unterseite zwei vom Kunststoff ummantelte Drahteinlagen 4 auf, die sich angenähert parallel zu und in geringem Abstand von dem Innenrand la bzw. Außenrand 1b der Beinschale 1 von deren Wadenbereich bis unter den Vorfuß-Schalenabschnitt 3 erstrecken und hier etwa im Bereich des Fersenbeines auslaufen.

Durch die Anordnung dieser vorzugsweise aus Federstahl bestehenden Drahteinlagen 4 ist es möglich, für die Beinschale 1 einen weichen, biegeelastischen Kunststoff zu wählen, so dass man eine autoadaptive Fußheberorthese erhält, die in dorsaler Fußbeugung einen geringen und in plantarer Fußbeugung einen hohen, die Fußund Schuhmasse kompensierenden Widerstand aufweist.

Zur Schonung des in der Zeichnung nicht näher dargestellten Schuhwerkes ist eine mittlere, im hinteren Fersenbereich der Beinschale 1 auf deren Rückseite angeformte Rippe 5 vorgesehen, die bei angelegtem Schuhwerk über dessen Schuhrand hinausragt.

## Patentansprüche

1. Fußheberorthese mit einer dorsalen, sich über den Rückfuß erstreckenden, aus Kunststoff bestehenden Beinschale (1), die über eine Gurtung (2) oder dergleichen an der Wade des Orthesenträgers festlegbar ist und sich mit einem mit ihr einteilig ausgebildeten Vorfuß-Schalenabschnitt (3) bis unter den Fußsohlenbereich erstreckt, **dadurch gekennzeichnet, dass** die Beinschale (1) in dorsaler Fußbeugung einen geringen und in plantarer Fußbeugung einen hohen, die Fußund Schuhmasse kompensierenden Widerstand aufweist und aus einem weichen, biegeelastischen Kunststoff, z.B. einem Low Density Polyethylen oder einem EVA, besteht, in den auf der Rück- bzw. Unterseite der Beinschale (1) zwei Federstahl-Drahteinlagen (4) eingebettet sind, die sich angenähert parallel zu und in geringem Abstand von dem Innen- bzw. Außenrand (1a, 1b) der Beinschale (1) von deren Wadenbereich bis unter den Vorfuß-Schalenabschnitt (3) erstrekken.

2. Fußheberorthese nach Anspruch 1, **dadurch gekennzeichnet, dass** die Drahteinlagen (4) etwa im unteren Bereich des Fersenbeines auslaufen.

3. Fußheberorthese nach Anspruch 1 oder 2, **dadurch gekennzeichnet, dass** die Drahteinlagen (4) nahezu vollständig vom Kunststoff umschlossen sind.

4. Fußheberorthese nach Anspruch 1, 2 oder 3, **gekennzeichnet durch** eine mittlere, im hinteren Fersenbereich der Beinschale (1) auf deren Rückseite angeformte Rippe (5), die bei angelegtem Schuhwerk über dessen Schuhrand hinausragt.

## Claims

1. Foot-lifter orthosis having a dorsal leg shell (1) which extends over the hind-foot, consists of plastic, can be secured in position on the calf of the orthosis-wearer via a strap (2) or the like and extends, with a fore-foot portion (3) of the shell, which portion is of one-piece construction with the said shell, right under the region of the sole of the foot, **characterised in that** the leg shell (1) has a resistance which is low in the case of dorsal flexure of the foot and high in the case of plantar flexure of the foot and which compensates for the mass of the foot and shoe, and the said leg shell consists of a soft, flexurally elastic plastic, for example a low-density polyethylene or an EVA, in which there are embedded, on the hind side and underside of the leg shell (1), two spring-steel wire inlays (4) which extend approximately parallel to, and at a slight distance from, the inner and outer edges (1a, 1b) of the said leg shell (1), from the calf region of the latter right under the fore-foot portion (3) of the said shell.

2. Foot-lifter orthosis according to claim 1, **characterised in that** the wire inlays (4) run out approximately in the lower region of the heel bone.

3. Foot-lifter orthosis according to claim 1 or 2, **characterised in that** the wire inlays (4) are almost completely enclosed by the plastic.

4. Foot-lifter orthosis according to claim 1, 2 or 3, **characterised by** a central rib (5) which is moulded on in the rear region of the heel of the leg shell (1), on the hind side of the latter, and which projects, when footwear is put on, above the edge of the shoe of the latter.

## Revendications

1. Orthèse pour pied tombant, comprenant une coque de jambe (1), dorsale, constituée de matière plastique, s'étendant sur l'arrière du pied, et qui peut être fixée au mollet du porteur d'orthèse par un sanglage (2) ou analogue, et qui s'étend jusque sous la zone de la plante du pied par une partie de la coque pour l'avant-pied (3), réalisée d'une seule pièce avec la coque de jambe, **caractérisée en ce que** la coque de jambe (1) présente en flexion dorsale du pied une faible résistance et en flexion plantaire du pied une forte résistance compensant la masse du pied et de la chaussure, et est constituée d'une matière plastique molle, élastiquement flexible, par exemple un polyéthylène faible densité ou un copolymère ethylène/acétate de vinyle, dans laquelle sont encastrés deux inserts en fil d'acier à ressort (4) sur le côté postérieur et respectivement inférieur de la coque de jambe (1), qui s'étendent sensiblement parallèlement aux bords intérieur et respectivement extérieur (1a, 1b) de la coque de jambe (1) et à faible distance de ceux-ci à partir de la zone du mollet de la coque de jambe (1) jusque sous la partie de la coque pour l'avant-pied (2).

2. Orthèse pour pied tombant selon la revendication 1, **caractérisée en ce que** les inserts en fil (4) se terminent à peu près dans la zone inférieure du calcanéum.

3. Orthèse pour pied tombant selon la revendication 1 ou 2, **caractérisée en ce que** les inserts en fil (4) sont pratiquement en totalité entourés de matière plastique.

4. Orthèse pour pied tombant selon la revendication 1, 2 ou 3, **caractérisée en ce qu'**une nervure médiane (5) formée sur le côté arrière de la zone postérieure du talon de la coque de jambe (1) dépasse du bord de la chaussure une fois la chaussure en place.
